# EUROPEAN PATENT APPLICATION

(11) **EP 2 957 229 A1**
(43) Date of publication of application: **23.12.2015**
(21) Application number: 14173337.8
(22) Date of filing: 20.06.2014
(51) Int. Cl.: A61B 5/087, A61B 5/097, A61B 5/091

(54) **Device for measuring pulmonary condition**

(71) Applicant: Spiromagic Aps, 2730 Herlev (DK)
(72) Inventor: Hansen, Martin Bo, 2730 Herlev (DK); Kjaer, Jørgen Ole, 2730 Herlev (DK); Sandholt, Jesper, 2730 Herlev (DK)
(74) Representative: Hoffmann Dragsted A/S

(57) **Abstract**

The present invention relates to a medical device for monitoring a physiological pulmonary condition of a user and comprising at least one mouthpiece and a transducer, wherein the at least one mouthpiece and the transducer are separate elements, and wherein the at least one mouthpiece is capable of and intended for being physically attached to and detached from the transducer, wherein the at least one mouthpiece, when attached to the transducer, is capable of passing at least part of a flow of exhaled air or inhaled air through the mouthpiece to the transducer, and wherein the transducer is capable of measuring the flow of exhaled air or inhaled air passed to the transducer and of providing data of the flow of exhaled air or inhaled air passed to the transducer. Also, the present invention relates to a mouthpiece for such medical device and a method and a system for monitoring a physiological pulmonary condition.

## Description

### Field of the invention

The present invention relates to a device for measuring and possibly recording the physiological pulmonary condition of a user of the device. The device comprises at least one mouthpiece and a transducer, wherein the mouthpiece is intended for the user to blow exhaled pulmonary air into or to draw inhaled air into. The invention also relates to a method for monitoring a physiological pulmonary condition of a user of the device. The invention also relates to a mouthpiece for such device and such method. Furthermore, the invention relates to a system for monitoring a physiological pulmonary condition of a user of the device.

### Background art

Persons suffering from a pulmonary disorder such as asthmatics or COPD (Chronic Obstructive Pulmonary Disease) may need medical attendance at a regular basis. The procedure is often that the person consults a private doctor or a hospital to get diagnosed and that a doctor or other specialist measures and possibly records the physiological pulmonary condition of the person. Such procedure is time-consuming, not only for the person, but also for the doctor or specialist.

Because the procedure is time-consuming, there is a risk that the physiological pulmonary condition of the patient is not measured at a regular basis, which may lead to irreparable deterioration of the physiological pulmonary condition of the person. Hence, there is a need for ensuring that the pulmonary condition of the person is measured regularly to possibly avoid irreparable damage.

US 8,034,002 discloses an electronic peak flow meter with software for executing a stochastic process control and evaluating the digital respiratory data collected by the meter. The programmed stochastic process control is capable of selecting and calculating any one of a number of pulmonary function parameters, including at least one statistical process control variable to determine a pulmonary threshold. Information systems linking doctor's offices and patient's meters are further provided to transmit warning data in cases where the pulmonary threshold is exceeded. US 8,034,002 discloses an electronically enhanced peak flow meter having electronics embedded for measuring the pulmonary capacity of the patient. Handling and other daily use of the mouthpiece may lead to the electronics being destroyed or at least the electronics, in time, making inaccurate measurements because of the use of the mouthpiece, such as spit covering the electronics, or the electronics being exposed to water or moist during daily use by the patient.

### Summary of the invention

It is an object of the present invention to wholly or partly overcome the above disadvantages and drawbacks of the prior art. More specifically, it is an object to provide an improved device, which provides an increased possibility of monitoring the physiological pulmonary condition of the user.

It is also an object of the invention to provide a device which provides a possibility of various users using the same transducer of the device, however, preferably not at the same time.

It is even an object of the invention to provide a device which increases general well-being of the user using the device, if the user uses the device over a prolonged period of time.

The above objects, together with numerous other objects, advantages, and features, which will become evident from the below description, are accomplished by a solution in accordance with the present invention comprising a medical device for monitoring a physiological pulmonary condition of a user and with at least one mouthpiece and a transducer,
- wherein the at least one mouthpiece and the transducer are separate elements, wherein the at least one mouthpiece is capable of and intended for being physically attached to and detached from the transducer,
- wherein the at least one mouthpiece, when attached to the transducer, is capable of passing at least part of a flow of exhaled air or inhaled air through the mouthpiece to the transducer, and
- wherein the transducer is capable of measuring one or more characteristics of the flow of exhaled air or inhaled air passed to the transducer and of providing data of the flow of exhaled air or inhaled air passed to the transducer.

The at least one mouthpiece and the transducer being separate devices provides the possibility of using the one and same transducer for different mouthpieces, either mouthpieces being different depending on the user of the mouthpiece, or the mouthpieces being different depending on the pulmonary condition of the user. Users having poorer pulmonary condition than other users may need a mouthpiece, where the pressure drop is less, when blowing into the mouthpiece, compared to other mouthpieces for users having better pulmonary condition. Therefore, the mouthpiece and the transducer are not uniquely linked, even if different mouthpieces are used with the one and same transducer. However, the different mouthpieces are not to be used at the same time together with the transducer. The mouthpiece will preferably be used only for passing flow of exhaled air or inhaled air to the transducer, not for measuring characteristics of the flow of exhaled air blown, or inhaled air drawn, by the user into the mouthpiece.

According to a preferred embodiment of a device according to the invention, the at least one mouthpiece is void of electronic elements exposed to the interior of the flow channel, such as the at least one mouthpiece being void of electronic elements for measuring characteristics of the flow of exhaled air or inhaled air passed to the transducer.

When the mouthpiece is void of electronic elements exposed to the interior of the flow channel, and therefore exposed to a surface of the mouthpiece, such as electronic elements for measuring characteristics of the flow of exhaled air or inhaled air passed to the transducer, the mouthpiece is capable of being cleaned, even in a dishwasher, without the risk of such electronic elements being destroyed during cleaning of the mouthpiece. Regular cleaning of the mouthpiece increases the general well-being of the user and provides a possibility of different users using the same mouthpiece, however, not at the same time.

According to possible embodiment of a device according to the invention,
- the mouthpiece is provided with a flow channel having a first orifice and a second orifice, the first orifice being intended for a user to blow a flow of exhaled air, or to draw a flow of inhaled air, into the flow channel and the second orifice being intended for discharge from the mouthpiece of the flow of exhaled air, or for intake to the flow channel of the flow of inhaled air, and
- wherein the mouthpiece is also provided with an air passage having a first orifice and a second orifice, the first orifice being fluidly connected with the flow channel and the second orifice being fluidly connected with a measuring means of the transducer, said air passage being capable of passing at least part of the flow of exhaled air or inhaled air from the flow channel to the transducer,
- wherein the flow channel is provided with a flow restriction in the flow channel, said flow restriction dimensioned dependent of the physiological pulmonary condition of the patient, and
- wherein the air passage is provided with a cross-sectional area dimensioned independently of the physiological pulmonary condition of the patient.

The mouthpiece, when provided with a flow channel and an air passage, provides a mouthpiece where the flow channel may be dimensioned depending on the pulmonary condition of the user of the device, and where the air passage is only for passing at least part of the flow of exhaled air or inhaled air to the transducer, independently of the user of the device. The air passage does not have any effect related to the pulmonary condition of the user, and the air passage needs no user-dependent calibration or user-dependent dimensioning.

In a preferred embodiment, the flow restriction is a selected size of a cross-sectional area of the flow channel. In possible alternative embodiments, the flow restriction is provided by a selected length of the flow channel for providing a pressure drop along the flow channel of the mouthpiece between a first orifice, where exhaled air enters the flow channel or inhaled air leaves the flow channel, and a second orifice, where exhaled air leaves the flow channel or inhaled air enters the flow channel.

Other means for providing a pressure drop along the flow channel of the mouthpiece between the first orifice and the second orifice, may be provided as alternative to, or in addition to, a certain size of the cross-sectional area and/or certain length of the flow channel for providing a pressure drop.

Other means for providing a pressure drop may be a spiral shaped flow channel having a curve of progression in two dimensions or in three dimensions of a Cartesian coordinate system. A cross-sectional area of a spiral shaped flow channel may either be constant along the line of progression or may change along the line of progression. Alternatively, or additionally, flow obstructions like an impeller or a venturi may be provided in the flow channel.

Geometrical shape of the flow channel may be selected depending on the design of the mouthpiece as such and of the medical device. Cross-section of the flow channel may be circular, oval, polygonal or a combination of these geometries.

Dimensions and geometrical shape of the flow channel may be provided already when manufacturing the mouthpiece, or may be provided as an add-in element to be placed in the mouthpiece as such, prior to use of the mouthpiece.

According to an embodiment of a device according to the invention, the mouthpiece has
- a first status in relation to the attachment to the transducer, in which first status measuring the flow of exhaled air or inhaled air passed to the transducer is disabled, and
- a second status in relation to the attachment to the transducer, in which second status measuring the flow of exhaled air or inhaled air passed to the transducer is enabled.

Providing a first status and a second status of the mouthpiece in relation to the transducer may be used for saving electrical power from e.g. batteries of the transducer, so that electrical power is only provided and only used when measuring is enabled.

According to an embodiment of a device according to the invention,
- the first status is a retracted position of the mouthpiece in relation to the transducer, and
- the second status is an extended position of the mouthpiece in relation to the transducer.

Also, in the retracted position, blowing of exhaled air or inhaled air through the mouthpiece is disabled, and in the extended position blowing of exhaled air or inhaled air through the mouthpiece is enabled.

Providing a first status disabling blowing through the mouthpiece, provision of electrical power and exclusion of electrical power from e.g. batteries of the transducer, is easy for the user to see and ensure, depending on whether the mouthpiece is in a state enabling blowing through the mouthpiece, or not.

According to an embodiment of a device according to the invention,
- the mouthpiece is attached to the transducer via a pivot joint, so that the mouthpiece is capable of pivoting in relation to the transducer,
- wherein the mouthpiece in a first pivoted position is in the retracted position in relation to the transducer, and
- wherein the mouthpiece in a second pivoted position is in the extended position in relation to the transducer.

A pivot joint is an easy and safe method of attaching the mouthpiece to the transducer, while at the same time providing a good possibility for the mouthpiece to be displaced from a first status to a second status, and vice versa.

According to a possible embodiment of a device according to the invention, the air passage extends along and parallel with the pivot axis of the pivot joint.

When the air passage extends along and parallel with the pivot axis, passing of flow of exhaled air or inhaled air from the flow channel to the transducer can be obtained in any angularly pivoted position of the mouthpiece in relation to the transducer.

According to a possible embodiment of a device according to the invention, the pivot joint is constituted by a sealing,
- wherein at least part of an outer circumference of the sealing constitutes a bearing between the mouthpiece and the transducer, and
- wherein a through-hole through the sealing, delimited by an inner circumference of the sealing, constitutes the air passage.

Moreover, the sealing may be at least partly made of elastomer or natural rubber.

A spring is provided for applying a force to the mouthpiece, maintaining the mouthpiece in the retracted position, said force to be overcome when displacing the mouthpiece from the retracted position to the extended position.

A sealing may function both as pivot joint and as air passage for passing flow of exhaled air or inhaled air from the flow channel to the transducer. Thereby, the one and same element, that is the sealing, provides two different functions necessary for the function of the embodiment of the device.

According to an embodiment of a device according to the invention, the device comprises at least a first mouthpiece, a second mouthpiece and a transducer, - wherein the first mouthpiece has a flow channel with a first cross-sectional area restriction and the second mouthpiece has a flow channel with a second cross-sectional area restriction, the cross-sectional areas at the restrictions viewed perpendicular to a longitudinal axis along a main flow direction of the flow channels, and wherein the cross-sectional area at the second cross-sectional area restriction is smaller than the cross-sectional area at the first cross-sectional area.

Providing at least two different mouthpieces, with different cross-sectional areas at cross-sectional area restrictions of the flow channel, makes it possible, either for the user to switch between the one or the other mouthpiece, or for the doctor or other specialist to provide to the one or the other user the first or the second mouthpiece, depending on the pulmonary condition of the user, and without having to consider which transducer to use, because the transducer functions with either one of the at least two mouthpieces.

According to a possible embodiment of a device according to the invention, - the transducer is capable of transmitting the data of the flow of exhaled air or inhaled air passed to the transducer further on to a remote data receiver, wherein the data are correlated with a patient for using the device, and wherein the remote data receiver is located by at least one of the following recipients of the data: a hospital medicating the patient, a doctor of the patient, a relative of the patient, the patient himself or herself.

Remote monitoring of the user at different locations, either at one location or the other location, or at different locations at the same time, such as both at the specialist and at relatives of the user, provides enhanced possibilities of assisting the user in remembering to perform regular measurements of the pulmonary condition and/or of detecting a deterioration or improvement of the pulmonary condition of the user.

According to a possible embodiment of a device according to the invention, the remote data receiver is at least one of the following data receivers: a mobile phone or cellular phone, a watch or other wearable, a tablet, a laptop.

The remote data receiver being a non-stationary device increases the possibility of the user herself or himself, a relative to the user or the doctor or other specialist, being capable of monitoring that the user performs regular measurements by using the device and possibly recording the measurements performed by the user.

According to a possible embodiment of a device according to the invention, outer boundaries of the at least one mouthpiece, in the retracted position, is enclosed within outer boundaries of the transducer, so that when the mouthpiece is attached to the transducer, and when the mouthpiece is in the retracted position, outer boundaries of the device are outer boundaries of the transducer.

When the device is not in use by the user, it is an advantage that the device does not take up more space than necessary. When not in use, the mouthpiece is not needed, and it is an advantage that the mouthpiece is wholly embedded in the transducer.

Objects of the invention are also accomplished by a method for monitoring a physiological pulmonary condition of a user of a device according to any of the preceding claims, said method comprising the steps of:
- displacing the mouthpiece from a retracted position to an extended position for measuring a flow of air exhaled or inhaled air by the user and blown or drawn into the mouthpiece,
- the user blowing air into a first orifice or drawing air into a second orifice of the mouthpiece, through a flow channel of the mouthpiece and through a second orifice or first orifice, respectively, of the mouthpiece,
- passing at least part of the flow or air from the flow channel of the mouthpiece to an air passage between the mouthpiece and the transducer,
- passing the flow of exhaled air or inhaled air from the air passage to measuring means of the transducer for measuring characteristics such as air pressure of the flow or air, and
- displaying data related to the characteristics of the flow of exhaled air or inhaled air, said data being displayed on the transducer and/or on a remote receiver.

According to as preferred aspect of the method according to the invention,
- first data of a first exhalation or inhalation and blowing of air or drawing of air into the mouthpiece is measured and recorded by the transducer and/or by a remote data receiver,
- wherein second data of a second exhalation or inhalation and blowing of air or drawing of air into the mouthpiece is measured and recorded by the transducer and/or by a remote data receiver,
- said first data and said second data being measured and recorded with a time interval between measuring the first data and the second data,
- wherein a possible change of data between the first data and the second data is measured so as to establish any difference between the first data and the second data, and
- wherein a possible change between the first data and the second data is used for establishing whether the physiological pulmonary condition of the user is getting worse or is getting better.

Objects of the invention are also accomplished by a system for monitoring a physiological pulmonary condition of a user, said system comprising:
- a device according to the invention,
- a data transmission channel between the device and a remote data receiver,
- a data recording means for recording data received by the remote data receiver,
- wherein the data transmission channel is at least one of the following wireless data transmission channel:
   WPAN such as Bluetooth, WLAN such as Wi-Fi, WMAN such as WiMAX and mobile/cellular networks,
- wherein the recording means is embedded in at least one of the following remote receivers:
   a mobile phone or cellular phone, a watch or other wearable, a tablet, a laptop, and
- wherein the recording means are able to record first data and second data different from the first data, and are provided with data displaying means for displaying the first data and the second data.

Transmitting and receiving data via wireless data transmission channels provide a good possibility of monitoring the user's pulmonary condition at different locations, either one location or the other location, or at different locations at the same time, such as both at the specialist and at relatives of the user. Also, by recording at least two sets of data, a trend in the pulmonary condition of the user may be monitored, to see whether the condition gets worse or gets better.

### Brief description of the drawings

The invention and its many advantages will be described in more detail below with reference to the accompanying schematic drawings, which for the purpose of illustration show some non-limiting embodiments and in which
Fig. 1 shows a system according to the invention for monitoring a physiologic pulmonary condition of a user,
Fig. 2 is a perspective view of an embodiment of the device for measuring a physiologic pulmonary condition of a user, and in which measuring is disabled,
Fig. 3 is a perspective view of the embodiment of a device for measuring a physiologic pulmonary condition of a user, and in which measuring is enabled,
Fig. 4 is a perspective, exploded view of the embodiment of the device,
Fig. 5a and fig. 5b are perspective views of the embodiment of a mouthpiece for a device according to the invention,
Fig. 6 is a plane, cross-sectional view of the device according to the invention, and
Fig. 7a-7d are plane cross-sectional views of various embodiments of a mouthpiece for a device according to the invention.

### Detailed description of the invention

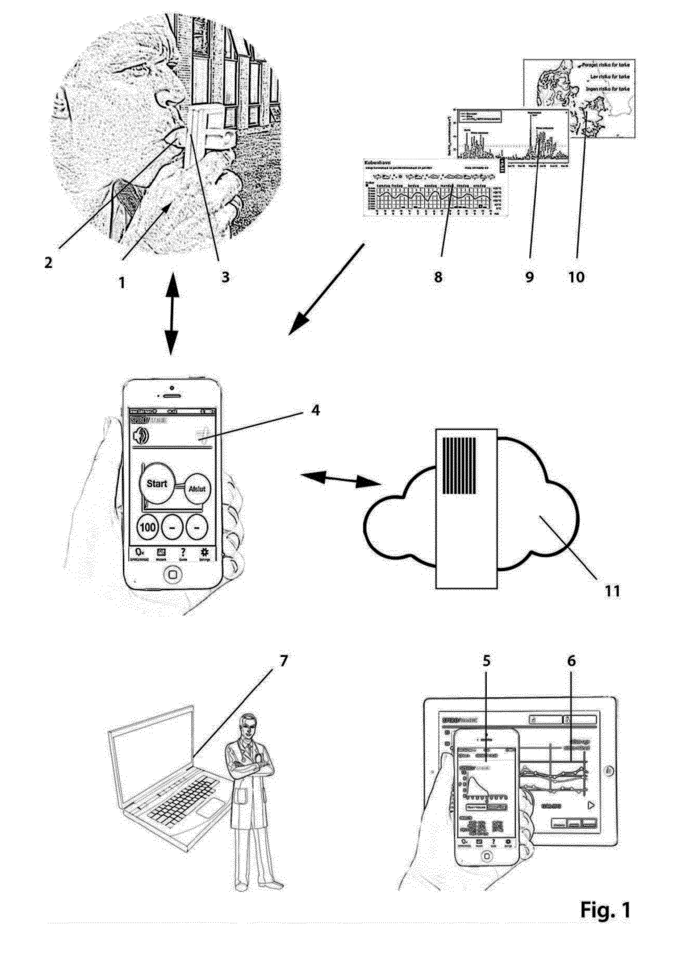

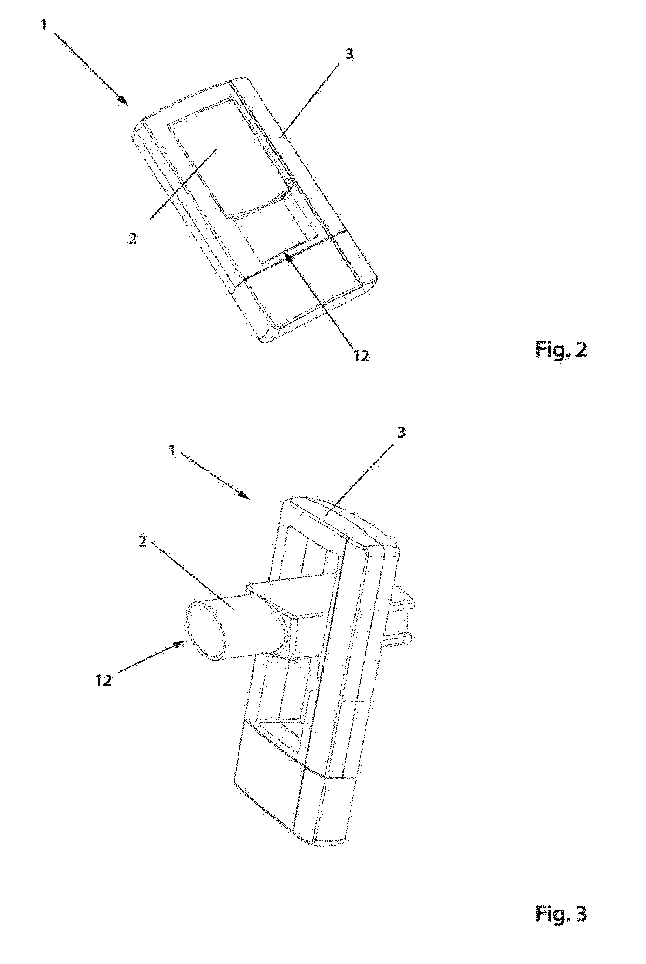

Fig. 1 shows a system for monitoring the physiological pulmonary condition of user. The system comprises a device 1 according to the invention. The device is to be used by a user exhaling air into a mouthpiece 2 of the device 1, creating a flow of exhaled air or inhaled air through the mouthpiece 2. One or more characteristics of at least part of said flow of exhaled air or inhaled air is to be measured by a transducer 3 of the device 1. The device 1 as such, and the function of the device 1 as such, is described in more detail with reference to figures 2 ff.

The system furthermore comprises a range of possible remote receivers of data transmitted by the transducer of the device, said data related to the one or more characteristics measured by the transducer 3. The range of possible remote receivers may comprise: a mobile or cellular phone 4 of the user, a mobile or cellular phone 5 or a tablet 6 of others than the user such as a doctor or other specialist or a relative of the user, or a laptop 7 or a tablet or a dongle of the user or of others than the user such as a doctor or other specialist or a relative of the user. The list of remote receivers is not exhaustive, other remote receivers known to the skilled person may be used.

Measurements by the transducer 3 of one or more characteristics of the flow of exhaled air or inhaled air through the mouthpiece 2 may be correlated with other data. Other data may be one or more of the following data: present and/or future weather conditions 8 in the environment of the user, rate of polluting particles 9 in the environment of the user, rate of pollen 10 in the environment of the user. The list of other data is not exhaustive, other data may be used, relevant for the measurements by the transducer 3 of one or more characteristics of the flow of exhaled air or inhaled air.

The other data may be data based on measurements performed simultaneously with measurement of the one or more characteristics of the flow of exhaled air or inhaled air. Alternatively or additionally, the data may be based on historic or empiric data at the location where the user is situated, when performing measurements of the exhaled air or inhaled air. Therefore, the transducer 3 may comprise means for establishing the position of the transducer 3, and therefore of the user, such as GPS (Global Positioning System).

The system comprises computer programs with algorithms for processing data transmitted by the transducer 3 to the one or more remote receivers 4-7. The computer programs are provided distant from the transducer 3 and distant from the remote receivers 4-7 such as so-called cloud computing 11. If the system comprises such distant computer programs, the transducer 3 and/or the remote receivers 4-7 comprise telecommunications means for communicating with the distant computer programs. Alternatively or additionally to using distant computer programs , computer programs and algorithms needed for processing the data transmitted by the transducer 3 is stored at one or more of the remote receivers 4-7.

The computer programs, software and algorithms, whether distant to the one or more of the remote receivers 4-7 or locally stored on one or more of the remote receivers 4-7, may have various features for displaying the measurements of the one or more characteristics of the flow of exhaled air or inhaled air. The measurement may be displayed according to one or more of the following options: displayed as a number being a percentage of the user's pulmonary capacity correlated with a reference pulmonary capacity; a graph showing the user's pulmonary capacity over a period of time during one exhalation of air or inhalation of air, possibly correlated with a reference pulmonary capacity; a graph showing the user's pulmonary capacity as a trend over a period of time covering at least two different exhalations of air or two different inhalations of air, possibly correlated with a reference pulmonary capacity, a graph showing the user's pulmonary capacity over a period of time during one exhalation of air or one inhalation of air, possibly correlated with a reference pulmonary capacity. The reference pulmonary capacity may a pulmonary capacity of a person not suffering from possible pulmonary incapacity as the user. The reference person is a person having the same gender and/or similar age and/or similar environmental conditions. The reference pulmonary capacity may in stead be a pulmonary capacity of the user during one or more earlier measurements of the pulmonary capacity of the user, for displaying a trend.

The remote receivers, apart from being capable to receive data from the transducer 3, may also be capable of processing, displaying, recording and/or storing the data. Depending on the type of remote receiver, depending on the user of the remote device and depending on for what purpose the remote receiver is receiving the data, the remote receiver may able to both display, process, record and store the data, or the remote receiver may be able to only perform some of the operations mentioned.

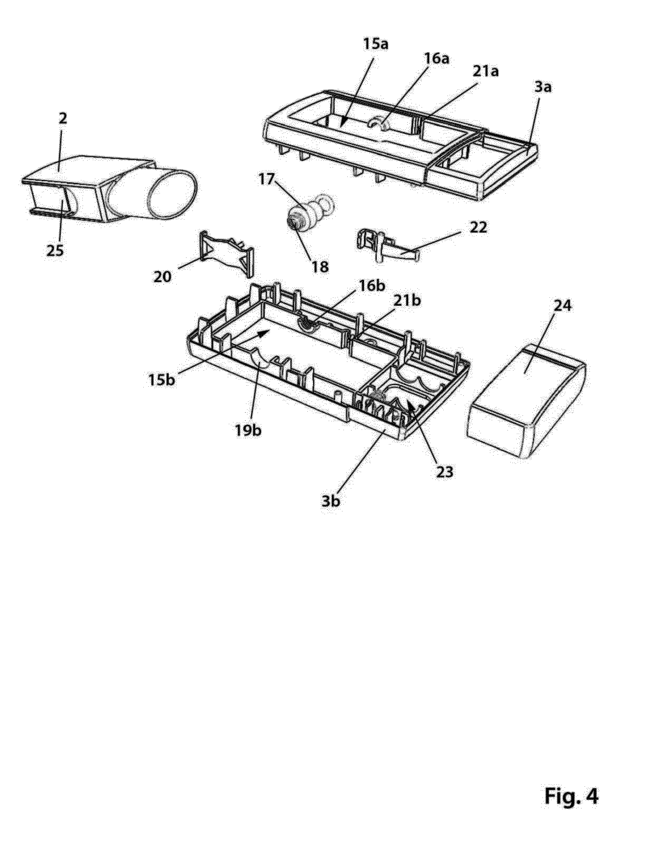

Fig. 2. shows a device for measuring one or more characteristics of a flow of air exhaled or inhaled air by a user. As mentioned, the device comprises a mouthpiece 2 and a transducer 3. The mouthpiece 2 is attached to the transducer 3 via a pivot joint (see fig. 4). The pivot joint of the mouthpiece 2 is releasable, thereby enabling the mouthpiece 2 to be detached from the transducer 3.

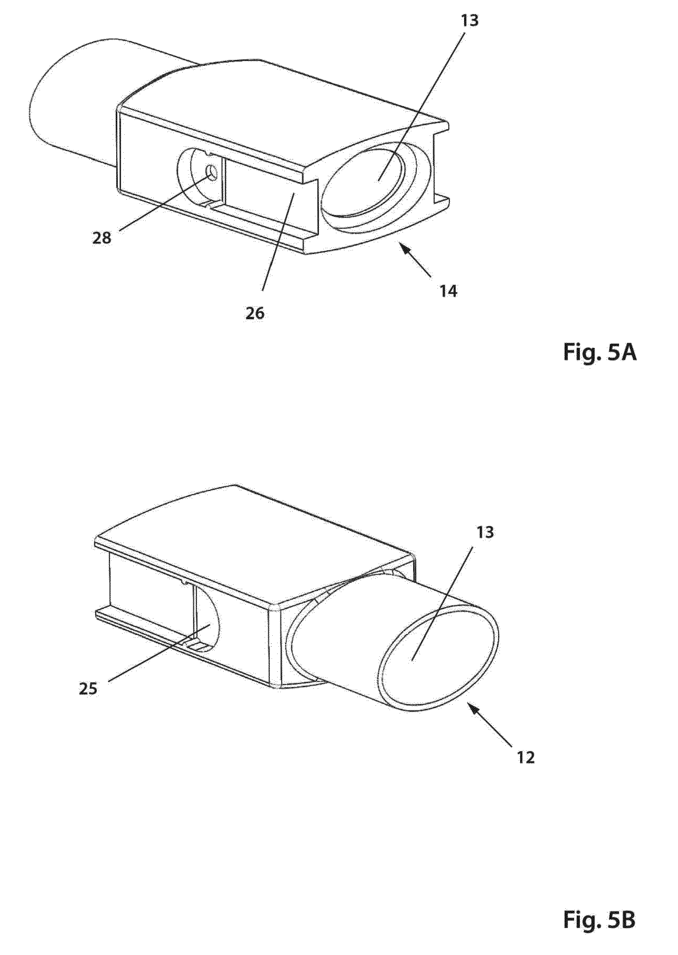

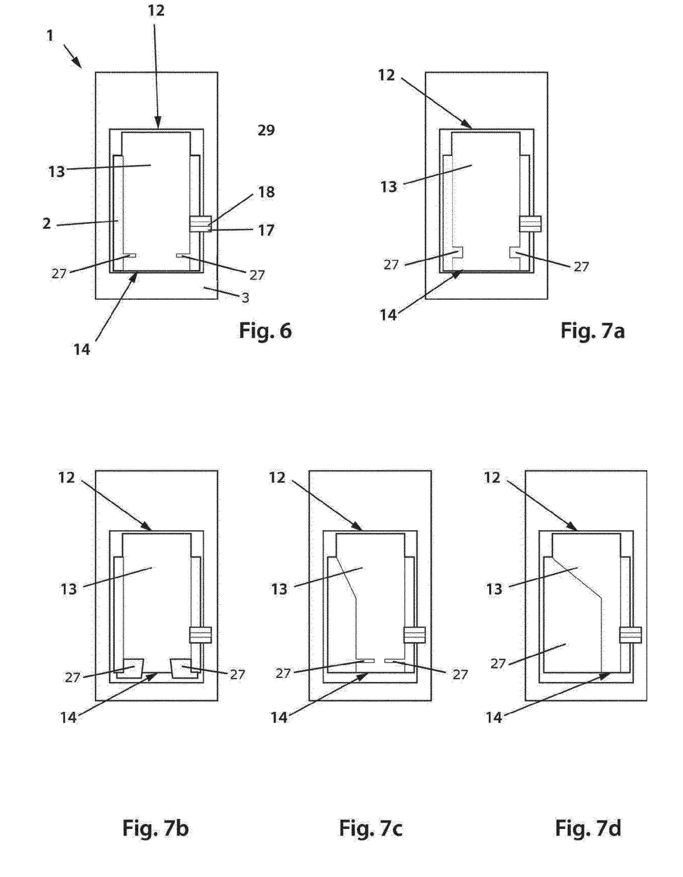

The mouthpiece 2 is provided with a first orifice 12 for a flow channel 13 (see fig. 5a-5b and figures 7a-7d) and a second orifice 14 of the flow channel 13. The first orifice 12 is for a user to blow exhaled air into or to draw inhaled air out of the mouthpiece 2. The second orifice 14 (see fig. 3B) is for the flow of exhaled air to leave the mouthpiece 2 or for the flow of inhaled air to enter the mouthpiece 2. An air passage 18 (see fig. 4) extends between the flow channel 13 of the mouthpiece 2 and the transducer 3. The transducer 3 is provided with electronics (not shown) for measuring one or more characteristics, such as a pneumatic pressure, of flow of air exhaled or inhaled by the user, blown by the user into the first orifice 12 and flowing through the flow channel 13 of mouthpiece 2.

In the figure shown, the mouthpiece 2 is pivoted to a position in relation to the transducer 3, where blowing of exhaled air, or drawing of inhaled air, into and through the flow channel 13 of the mouthpiece 2 is disabled. The position constitutes a first status of the mouthpiece 2, where the mouthpiece 2 is in a retracted position in relation to the transducer 3. When the mouthpiece 2 is in the first, retracted status in relation to the transducer 3, measurement by the transducer 3 of one or more characteristics of the flow of air is disabled. Electric power from batteries or other source of electrical power is thereby saved, when blowing of exhaled air, or drawing of inhaled air, through the mouthpiece 2 is disabled, and where corresponding measurements of the flow of air therefore is not needed.

Furthermore, in the first, retracted status of the mouthpiece 2 in relation to the transducer 3, where blowing of exhaled air, or drawing of inhaled air, into and through the flow channel 13 of the mouthpiece 2 is disabled, outer boundaries of the mouthpiece 2 is wholly enclosed within outer boundaries of the transducer 3 resulting in the device as a whole does not take of more space than the transducer 3 itself.

Fig 3. shows the embodiment of a device being the same embodiment as shown in Fig. 2. As mentioned, the device comprises the mouthpiece 2 and a transducer 3. The mouthpiece 2 is attached to the transducer 3 via a pivot joint (see fig. 4). The pivot joint of the mouthpiece 2 is releasable, thereby enabling the mouthpiece 2 to be detached from the transducer 3.

In the figure shown, the mouthpiece 2 is pivoted to a position in relation to the transducer 3, where blowing of exhaled air, or drawing of inhaled air, into and through the flow channel 13 of the mouthpiece 2 is enabled. The position constitutes a second status of the mouthpiece, where the mouthpiece is in an extended position in relation to the transducer 3. When the mouthpiece 2 is in the second, extended status in relation to the transducer 3, measurement by the transducer 3 of one or more characteristics of the flow of air is enabled.

The mouthpiece 2 is preferably void of any electronics for measuring, transmitting, receiving or recoding of data related to the flow of exhaled air or inhaled air through flow channel 13 of the mouthpiece 2. Thereby, the mouthpiece 2, when detached from the transducer 3, can easily be cleaned by the user, either by hand in tap water or in a dish washer, without risk of any electronics being damaged during cleaning of the mouthpiece 2. Thereby, the general wellbeing of the user of the device is increased due to possibility of reducing risk of bacteria collecting on the mouthpiece 2. Also, by providing possibility of easy cleaning of the device, the mouthpiece 2 may be used by different users without risk of bacteria from one user being passed on to other users.

The transducer 3 is provided with electronics and electrical power for measuring one or more characteristics of the flow of exhaled air or inhaled air through flow channel 13 of the mouthpiece 2. The electronics may be one or more pressure sensors, one or more flow sensors or other sensors for measuring characteristics of the flow of exhaled air or inhaled air through the mouthpiece 2. The transducer 3 is also provided with means for transmitting data of the measurements to a remote receiver. Data is preferably transmitted via wireless data transmission such as Bluetooth. Data is also preferably transmitted to a mobile or cellular phone, which phone has computer programs for processing the data received (see fig. 1). The mobile or cellular phone may also have hardware and computer programs for passing on data to other remote devices, either a remote receiver at a doctor or other specialist, or a remote receiver at the user herself or himself or at relatives of the user.

Fig. 4 shows a perspective view of the device in an exploded view. The transducer 3 is constituted by a first part 3a and a second part 3b to be mutually joined. The first part 3a and the second part 3b of the transducer 3 are provided with cavities 15a and 15b for taking up the mouthpiece 2. The cavities 15a,15b are provided with first recesses 16a,16b for accommodating a rubber sealing 17 comprising an air passage 18. The transducer 3 is also provided with a second recess 19a,19b (only second recess 19b visible and shown) for accommodating a pivot bearing 20. The first part 3a and the second part 3b of the transducer 3 are furthermore provided with third recesses 21a,21b for accommodating a springlike element 22 for maintaining the mouthpiece 2 in a retracted position as shown in Fig. 2, when the device is not in use. For displacing the mouthpiece 2 from the first, retracted status shown in fig. 2 to the second, extended status shown in fig. 3, a spring force from the element 21 must be overcome.

The transducer 3 is also provided with a fourth recess 23 and a cover 24 for the fourth recess 23, for accommodating batteries providing electric power to electronics (not shown) inside the transducer 3. The electronics are for measuring one or more characteristics of the flow of exhaled air or inhaled air and for transmitting data of the measured flow of exhaled air or inhaled air to one or more remote receivers 4-7 (see fig. 1) of the data.

The mouthpiece 2 is moulded in one piece provided with slots 25, 26 (only one shown, see also fig. 5a and fig. 5b) on each side of the mouthpiece 2 for attaching the mouthpiece 2 to the transducer 3 by sliding the slots along the one pivot bearing constituted by the rubber sealing 17 and the pivot bearing 20, opposite to the rubber sealing 17. When the mouthpiece 2 has been attached to the transducer 3 by means of the rubber sealing 17 and the pivot bearing 20, the air passage 18 provided in the rubber sealing 17 is in line with a corresponding hole 28 (not shown, see fig. 5a) in the one side of the mouthpiece 2. Thereby, at least part of flow of exhaled air or inhaled air through the flow channel 13 of the mouthpiece 2 is passed to the transducer 3 through the air passage 18.

The mouthpiece 2 may be provided with a marking (not shown) at a surface of the mouthpiece 2 for showing to the user at which side of the mouthpiece the hole 28 (not shown, see fig. 5a) is provided. Alternatively, a hole may be provided in each side of the mouthpiece 2, the one communicating with the air passage 18 and the other hole being blocked by the pivot bearing 20. Thereby, the mouthpiece 2 may be attached to the transducer 3 without notice to which side a hole to be aligned with the air passage 18 is provided.

Fig. 5a and fig. 5b show perspective views of the mouthpiece 2 according to the embodiment shown in fig. 2 and fig. 3. As mentioned, the mouthpiece 2 has a flow channel 13 with a first orifice 12 for blowing of exhaled air or inhaled air by the user into the flow channel 13 and a second orifice 14 for the exhaled or inhaled flow of air to leave the mouthpiece 2. The mouthpiece 2 also has a hole 28, see fig. 5a, passing at least part of the flow of exhaled air or inhaled air from the flow channel 13 to the transducer 3 (see also fig. 4).

Fig. 6 is a schematic view of the device shown in fig. 2-4. The device comprises a mouthpiece 2 and a transducer 3. The mouthpiece 2 is detachably attached to the transducer 3 via a pivot joint having a pivot bearing (see fig. 4) at least at one side of the mouthpiece 2, and a rubber sealing 17 at the other side of the mouthpiece 2.

In the figure shown, the mouthpiece 2 is in the first status, that is the retracted position in relation to the transducer 3, and where blowing of exhaled air or inhaled air through the flow channel 13 between the first orifice 12 and the second orifice 14 is disabled, and where measurement of one or more characteristics of a flow of air therefore is disabled, to save battery power or other electrical power for the electronics enclosed in the transducer 3.

The air passage 18 extends through the rubber sealing (see also fig. 4) from the flow channel 13 of the mouthpiece 2 to the electronics 29 embedded in the transducer 3. The air passage 18 extends along and parallel with a longitudinal axis of the rubber sealing 17, and also functioning as pivot bearing at one side of the mouthpiece 2. Thereby, the air passage 18 is capable of passing at least part of the flow of exhaled air or inhaled air from the flow channel 13 to the transducer 3 in any angularly pivoted position of the mouthpiece 2 in relation to the transducer 3, apart from the position shown, where the mouthpiece 2 is in the first, retracted status in relation to the transducer 3.

Figs. 7a-7d show different cross-sections of the flow channel 13 through the mouthpiece 2. Different users of a device according to the invention have different pulmonary capacities. The cross-sectional area of the flow channel 13 is preferably dimensioned according to the pulmonary capacity of the user. Users having a relatively large pulmonary capacity are capable of blowing exhaled air, or capable of drawing inhaled air, through a smaller cross-sectional area than users having a relatively small pulmonary capacity.

If the pulmonary capacity of the user is very small compared to average pulmonary capacity of people, the cross-sectional area of the flow channel 13 is small as shown in fig. 7c-7d. If the pulmonary capacity of the user is moderate compared to average pulmonary capacity of people, the cross-sectional area of the flow channel 13 is smaller, as shown in fig. 7b, than the cross-sectional area shown in fig. 7a. If the pulmonary capacity of the user is similar to average pulmonary capacity of people, the cross-sectional area of the flow channel 13 is large, as shown in fig. 7a, than the cross-sectional area shown in fig. 7b and as shown in fig. 7c and 7d.

In the embodiments shown, the cross-sectional area of the flow channel 13 of the mouthpiece 2 is delimited by different types and shapes of one or more obstructions 27 provided in the flow channel 13. The one or obstructions 27 provided may extend from an inner side wall in itself extending longitudinally straight along the flow channel 13 through the mouthpiece 2. Alternatively, or additionally, the one or more obstructions mat be a change of the longitudinal extension of the sidewall itself of the flow channel 13 through the mouthpiece 2.

Common to the different mouthpieces 2 with different cross-sectional areas of the flow channel 13 between the first orifice 12 and the second orifice 14, as shown in fig. 7a-7d, is that each of the different mouthpieces 2 may be used in the one and same transducer 3. Therefore, the device only has to be calibrated for the specific user by selecting the one or the other mouthpiece 2. The transducer 3 may have means for registering, whether it is the one or other mouthpiece 2 with the one or the other cross-sectional area of the flow channel 13, which is attached to the transducer 3. Means for registering which mouthpiece 2 is attached to the transducer 3 may comprise: Manual input in the transducer 3 such as a selector with, e.g., three possible selections, or automatic input in the transducer 3 such as an RFID-tag embedded in the mouthpiece 2 and a corresponding RFID reader in the transducer 3.

All the figures are highly schematic and not necessarily to scale, and they show only those parts which are necessary in order to elucidate the invention, other parts being omitted or merely suggested.

Although the invention has been described in the above in connection with preferred embodiments of the invention, it will be evident for a person skilled in the art that several modifications are conceivable without departing from the invention as defined by the following claims.

## Claims

1. Medical device for monitoring a physiological pulmonary condition of a user and comprising at least one mouthpiece and a transducer,
- wherein the at least one mouthpiece and the transducer are separate elements, and wherein the at least one mouthpiece is capable of and intended for being physically attached to and detached from the transducer,
- wherein the at least one mouthpiece, when attached to the transducer, is capable of passing at least part of a flow of exhaled or inhaled air through the mouthpiece to the transducer, and
- wherein the transducer is capable of measuring one or more characteristics of the flow of exhaled or inhaled air passed to the transducer and of providing data of the flow of exhaled air passed to the transducer.

2. Medical device according to claim 1,
- wherein the at least one mouthpiece is void of electronic elements having physical contact with the interior of the flow channel, such as void of electronic elements for measuring characteristics of the flow of exhaled air or inhaled air passed to the transducer.

3. Medical device according to claim 1 or 2,
- wherein the mouthpiece is provided with a flow channel having a first orifice and a second orifice, the first orifice being intended for a user to blow a flow of exhaled air or to draw a flow of inhaled air into the mouthpiece and the second orifice being intended for discharge from the mouthpiece of the flow of exhaled air or inhaled air from the mouthpiece, and
- wherein the mouthpiece is also provided with an air passage having an inlet and an outlet, the inlet being fluidly connected with the flow channel and the outlet being fluidly connected with a measuring means of the transducer, said air passage being capable of passing at least part of the flow of exhaled air or inhaled air from the flow channel to the transducer, and
- wherein the flow channel is provided with at least one of a selected dimension and selected a geometrical shape and a selected size, preferably having a selected cross-sectional area, dimensioned based upon the physiological pulmonary condition of the user.

4. Medical device according to any of claims 1-3, wherein the at least one mouthpiece has
- a first status in relation to the attachment to the transducer, in which first status measuring the flow of exhaled air or inhaled air passed to the transducer is disabled, and
- a second status in relation to the attachment to the transducer, in which second status measuring the flow of exhaled air or inhaled passed to the transducer is enabled.

5. Medical device according to claim 4,
- wherein the first status is a retracted position of the mouthpiece in relation to the transducer, and
- wherein the second status is an extended position of the mouthpiece in relation to the transducer.

6. Medical device according to claim 5,
- wherein the mouthpiece is attached to the transducer via a pivot joint, so that the mouthpiece is capable of pivoting in relation to the transducer,
- wherein the mouthpiece in a first pivoted position is in the retracted position in relation to the transducer, and
- wherein the mouthpiece in a second pivoted position is in the extended position in relation to the transducer.

7. Medical device according to claim 3 and 6, wherein the air passage extends along and parallel with the pivot axis of the pivot joint.

8. Medical device according to claim 6 or 7, wherein the pivot joint is a sealing,
- wherein at least part of an outer circumference of the sealing constitutes a bearing between the mouthpiece and the transducer, and
- wherein a through-hole through the sealing, delimited by an inner circumference of the sealing, constitutes the air passage.

9. Medical device according to any of claims 1-8 and comprising at least a first mouthpiece, a second mouthpiece and a transducer,
- wherein the first mouthpiece has a flow channel with a first cross-sectional area and the second mouthpiece has a flow channel with a second cross-sectional area, the first and the second cross-sectional areas viewed perpendicular to a longitudinal axis along a main flow direction of the flow channels, and wherein the second cross-sectional area is smaller than the first cross-sectional area.

10. Medical device according to any of claims 1-9,
- wherein the transducer is capable of transmitting the data of the flow of exhaled air or inhaled air passed to the transducer further on to a remote data receiver, wherein the data are correlated with a user of the device, and
- wherein the remote data receiver is located by at least one of the following recipients of the data: a hospital medicating the user, a doctor of the user, a relative of the user, the user herself or himself.

11. Medical device according to any of claim 1-10, wherein outer boundaries of the at least one mouthpiece, in the retracted position, is enclosed within outer boundaries of the transducer, so that when the mouthpiece is attached to the transducer, and when the mouthpiece is in the retracted position, outer boundaries of the device are outer boundaries of the transducer.

12. A method for monitoring a physiological pulmonary condition of a user of a device according to any of the preceding claims, said method comprising the steps of:
- displacing the mouthpiece from a retracted position to an extended position for measuring a flow of air exhaled or inhaled by the user and blown or drawn into the mouthpiece,
- the user blowing air into a first orifice of the mouthpiece, through a flow channel of the mouthpiece and through a second orifice of the mouthpiece,
- passing at least part of the flow or air from the flow channel of the mouthpiece to an air passage between the mouthpiece and the transducer,
- passing the flow of exhaled air or inhaled air from the air passage to a measuring means of the transducer for measuring characteristics such as air pressure of the flow or air, and
- displaying data related the characteristics of the flow of exhaled air or inhaled air, said data being displayed on the transducer and/or on a remote the receiver.

13. A method according to claim 12,
- wherein first data of a first exhalation or inhalation and blowing of air or drawing of air into the mouthpiece is measured and recorded by the transducer and/or by a remote data receiver,
- wherein second data of a second exhalation or inhalation and blowing of air or drawing of air into the mouthpiece is measured and recorded by the transducer and/or by a remote data receiver,
- said first data and said second data being measured and recorded with a time interval between measuring the first data and the second data,
- wherein a possible change of data between the first data and the second data is measured so as to establish any difference between the first data and the second data, and
- wherein a possible change between the first data and the second data is used for establishing whether the physiological pulmonary condition of the user is getting worse or is getting better.

14. A mouthpiece for a medical device according to any of claims 1-11,
- wherein the mouthpiece is provided with a flow channel for a user to blow exhaled air into or to draw inhaled air into,
- wherein the mouthpiece is void of electronic elements exposed to an interior of the flow channel, with the proviso that said electronic elements are for measuring characteristics of the flow of exhaled air or inhaled air, and
- wherein the mouthpiece is provided with at least one air passage for passing of at least part of the flow of exhaled air or inhaled air from the flow channel of the mouthpiece, out of the mouthpiece, and to an exterior transducer for measuring characteristics of the flow of exhaled air or inhaled air.

15. A system for monitoring a physiological pulmonary condition of a user, said system comprising:
- a device according to any of claims 1-11,
- a data transmission channel between the device and a remote data receiver,
- a data recording means for recording data received by the remote data receiver
- wherein the data transmission channel is at least one of the following wireless data transmission channels:
WPAN such as Bluetooth, WLAN such as Wi-Fi, WMAN such as WiMAX and mobile/cellular networks,
- wherein the recording means is embedded in at least one of the following remote receivers: a mobile phone or cellular phone, a watch or other wearable, a tablet, a laptop, and
- wherein the recording means are capable of recording first data and second data different from the first data, and are provided with data displaying means for displaying the first data and the second data.
